# EUROPEAN PATENT APPLICATION

(11) **EP 2 586 358 A1**
(43) Date of publication of application: **01.05.2013**
(21) Application number: 11798223.1
(22) Date of filing: 23.06.2011
(51) Int. Cl.: A61B 1/00, G02B 23/24, G02B 23/26

(54) **PROBE, DIAGNOSIS DEVICE, AND METHOD FOR USING THE DIAGNOSIS DEVICE**

(30) Priority: 25.06.2010 JP 2010145296
(71) Applicant: Konica Minolta Advanced Layers, Inc., Hachioji-shi, Tokyo 192-8505 (JP)
(72) Inventor: OHZAWA, Soh, Hachioji-shi Tokyo 192-8505 (JP); NATSUNO, Yasuyuki, Hachioji-shi Tokyo 192-8505 (JP); FUJIWARA, Katsumi, Hachioji-shi Tokyo 192-8505 (JP); ATARASHI, Yuichi, Hachioji-shi Tokyo 192-8505 (JP)
(74) Representative: Henkel, Breuer & Partner
(86) International application number: PCT/JP2011/064429
(87) International publication number: WO 2011/162342

(57) **Abstract**

A probe (4) is provided with at least a light projecting optical fiber (9) and a light receiving optical fiber (10) and is configured so that excitation light guided by the light projecting optical fiber is applied to the portion of a living organism which is to be observed and then light emitted, due to the excitation light, from the portion to be observed is received by the light receiving optical fiber. The probe (4) is also provided with an illumination means used for the capture of an image by the image capturing means of an endoscope body. The illumination means is, for example, a light emitting diode which is provided to the front end of the probe, a light guiding optical fiber (11) which guides illumination light from an illumination light source (5c) to the front end of the probe, or a light emitting substance piece which is disposed at the front end of the probe and emits white fluorescent light by excitation.

## Description

### Technical Field

The present invention relates to a probe inserted through an endoscope channel, and a probe which includes an optical system which irradiates irradiated light on an observation target region of biological tissue and which receives emitted light emitted from the observation target region due to the irradiated light.

### Background Art

Today, as an upper esophageal endoscope, an oral type is widely used and a nasal type is also becoming widely used.
Lately, other than the widely-called endoscope video scope, various special diagnosis devices which employ optical principle such as an ultrasonic diagnosis device, a fluorescence diagnosis device, and the like are proposed, and some are in practical use.
Especially, a fluorescence diagnosis device which applies fluorescence is much anticipated because the fluorescence diagnosis device obtains invisible information which cannot be obtained by the endoscope video scope and is useful for diagnosis to achieve early detection of malignant tumor.
As a diagnosis tool for such diagnosis, in other words, a probe, there is a probe which reaches inside the body through a forceps channel of the endoscope or a probe which is formed as one with the endoscope.
Here, the forceps channel is a tunnel shaped path formed inside the endoscope from a base end to a tip end of the endoscope to pass through a treatment tool such as a forceps or capturing net. The forceps channel is also called a work channel or inserting channel (channel may be noted as channel). Below, the above described tunnel shaped path formed inside the endoscope from the base end to the tip end of the endoscope is to be the endoscope channel.

The oral type endoscope has an outer diameter of about 10 mm, and most include an endoscope channel about slightly less than 3 mm.
When a probe is inserted through such endoscope channel, the conventional endoscope can be used and the probe enters the internal body lumen through a relatively gentle curve. Although flexibility as in a nasal type endoscope is not demanded, an outer diameter which is very small to pass through the endoscope channel is necessary. Therefore, depending on the configuration on which the probe is mounted, the configuration tends to be highly precise.
A lower gastrointestinal endoscope is inserted from an anus for diagnosis of large intestine starting from a rectum. The lower gastrointestinal endoscope similarly includes an endoscope channel, and is common with the oral type endoscope in that the endoscope channel can be used to insert and use the diagnosis equipment and probe.

Lately, there are needs to overlap the result detected through the probe to an image such as the esophageal inner wall or gastric wall imaged by the endoscope main body to be useful for diagnosis. By overlapping the result detected through the probe such as fluorescence intensity, etc. to a normal image imaged by the endoscope main body, a doctor, patient, etc. can identify a lesion which cannot be identified visually together with the position on the normal image.
Generally, the endoscope is a straight view type which images in a direction of movement of the endoscope. For example, when the probe is a side view type which observes the side perpendicular to the direction of movement of the endoscope, the observation target region of the probe is outside the field of view of the endoscope. Therefore, it is difficult to overlap the result detected through the probe to a normal image imaged by the endoscope main body.
Therefore, there are needs to match the observation direction of the probe employed in the endoscope with the imaging direction of the endoscope main body.

The Patent Documents 1 to 4 describe a probe inserted through an endoscope channel.
The probe described in Patent Document 1 uses the same optical fiber for transmitting light and receiving light. With such technique, the optical path is switched using a half mirror. Therefore, the quantity of light which is lost is large and reliability of diagnosis decreases. If the quantity of light is increased, there is a concern of bad influence on the human body, and there is a limit with a configuration which uses a single optical path for both transmitting light and receiving light.
The probe described in Patent Documents 2 to 4 include optical fiber for transmitting excitation light and does not include optical fiber for receiving light emitted from the lesion section, and observation of fluorescence, etc. is performed through the imaging section of the endoscope main body. Paragraph 0024 of Patent Document 4 describes the power source supply to the optical source section for normal observation is stopped manually in order to suitably observe the fluorescence.

### Prior Art Document

### Patent Document

Patent Document 1: Japanese Patent Application Laid-Open Publication No. 2000-88929
Patent Document 2: Japanese Patent Application Laid-Open Publication No. 2006-198106
Patent Document 3: Japanese Patent Application Laid-Open Publication No. 2007-14633
Patent Document 4: Japanese Patent Application Laid-Open Publication No. 2010-104391

### Disclosure of the Invention

### Problems to be Solved by the Invention

However, in the above prior art, there are problems as described below.
Illumination is essential for obtaining an image with the endoscope main body.
When the probe inserted through the endoscope channel is a probe which uses optical principles, there is a problem that the illumination included in the endoscope becomes a disturbance, and suitable measurement (diagnosis) cannot be performed.
Therefore, when observation is performed through the probe, there is a necessity to take measures such as to turn off the illumination of the endoscope main body or to shield the target region to be observed by the probe from light. In order to further obtain an image by the endoscope main body afterward, the illumination turned off needs to be turned on or the shield from light needs to be removed. With this, there is a possibility that the operation of the endoscope main body and the special diagnosis device becomes complicated. As a result, there is a possibility that the examination time becomes long and physical and mental burden of the patient increases. Therefore, the operator manually switching the illumination of the endoscope main body between on and off as described in Patent Document 4 is not desirable.

The present invention has been made in consideration of the above problems of the conventional technique, and the object is to provide a probe inserted through an endoscope channel and to provide a probe which can perform both the imaging by the endoscope main body and the observation by the probe with preferable operability and observation properties.

### Means for Solving the Problem

In order to achieve the above object, the invention of claim 1 is a probe inserted through an endoscope channel comprising at least:
a transmitting light optical fiber; and
a receiving light optical fiber,
wherein excitation light guided by the transmitting light optical fiber is irradiated to an observation target region of biological tissue and emitted light emitted from the observation target region due to the excitation light is received to be guided to a base end side of the probe by the receiving light optical fiber,
the prove further comprising an illuminating section to be used in imaging by an imaging section of an endoscope main body.

The invention of claim 2 is the probe of claim 1, further comprising a light guiding lens which guides the excitation light and the emitted light to a tip end side of the transmitting light optical fiber and the receiving light optical fiber.

The invention of claim 3 is the probe of claim 2, wherein the light guiding lens is a light condensing lens.

The invention of claim 4 is the probe of claim 2, wherein the light guiding lens is a collimated lens.

The invention of claim 5 is the probe of claim 2, further comprising a tube with water shielding properties to form an outer circumferential wall, wherein a tip end opening of the tube is sealed to be waterproof by the light guiding lens.

The invention of claim 6 is the probe of claim 1, further comprising a tube with water shielding properties to form an outer circumferential wall, wherein a tip end opening of the tube is sealed to be waterproof by a cover member.

The invention of claim 7 is the probe of claim 1, further comprising a tube with water shielding properties to form an outer circumferential wall, wherein a tip end face of the transmitting light optical fiber and the receiving light optical fiber is exposed to a probe tip end and a space between the tip end section of the transmitting light optical fiber and the receiving light optical fiber and the tube is sealed to be waterproof.

The invention of claim 8 is the probe of any one of claims 1 to 7, further comprising a light emitting diode provided at a probe tip end section as the illuminating section.

The invention of claim 9 is the probe of any one of claims 1 to 7, further comprising illuminating light guiding optical fiber which guides illuminating light from an illuminating light source to a probe tip end section as the illuminating section.

The invention of claim 10 is the probe of any one of claims 1 to 7, further comprising as the illuminating section, a light emitting material piece which is provided at a probe tip end section and which emits white color fluorescence by excitation, and a section which irradiates excitation light to the light emitting material piece.

The invention of claim 11 is the probe of claim 10, further comprising a light emitting diode provided adjacent to the light emitting material piece as the section which irradiates excitation light to the light emitting material piece.

The invention of claim 12 is the probe of claim 10, further comprising optical fiber which guides excitation light from a light source to the light emitting material piece as the section which irradiates excitation light to the light emitting material piece.

The invention of claim 13 is the probe of claim 2, further comprising illuminating light guiding optical fiber which guides illuminating light from an illuminating light source to a probe tip end section as the illuminating section, wherein the illuminating light guiding optical fiber is provided so that an output light end faces an outer circumferential section of the light guiding lens to allow the guided illuminating light to enter the outer circumferential section of the light guiding lens.

The invention of claim 14 is the probe of claim 2, wherein a flange section is formed outside the effective diameter of the light guiding lens, and the probe further comprises illuminating light guiding optical fiber which guides illuminating light from an illuminating light source to a probe tip end section as the illuminating section, wherein the illuminating light guiding optical fiber is provided so that an output light end faces the flange section to allow the guided illuminating light to enter the flange section.

The invention of claim 15 is the probe of claim 2, wherein a circumferential groove is formed in a circular shape in the flange section; and the output light end section of the illuminating light guiding optical fiber is inserted in the circumferential groove.

The invention of claim 16 is the probe of either one of claim 14 or claim 15, wherein a diffusing section which diffuses illuminating light is formed in the flange section and the output light end of the illuminating light guiding optical fiber is provided to be in contact with the flange section.

The invention of claim 17 is the probe of either one of claim 14 or claim 15, further comprising a diffusing plate which is provided between the flange section and the output light end of the illuminating light guiding optical fiber and which diffuses illuminating light, wherein the output light end of the illuminating light guiding optical fiber is provided to be in contact with the diffusing plate.

The invention of claim 18 is the probe of any one of claims 14 to 17, wherein the output light end of the illuminating light guiding optical fiber is provided so as to be inclined outward and a facing face of the flange section facing the output light end is formed so as to be inclined inward.

The invention of claim 19 is the probe of any one of claims 14 to 18, wherein an illuminating lens which corresponds to each one of the illuminating light guiding optical fiber and which diffuses the illuminating light is formed in the flange section.

The invention of claim 20 is the probe of claim 19, further comprising a plurality of the illuminating lenses wherein the optical axes of the illuminating lenses are provided on a same concentric circle.

The invention of claim 21 is the probe of claim 2, wherein an outer shape of the light guiding lens is formed in a D shape where a portion is missing from a round shape and an electric line or optical fiber composing the illuminating section is passed through a space corresponding to the missing section.

The invention of claim 22 is the probe of any one of claims 1 to 7, further comprising a holding member which holds the optical fiber composing the illuminating section, the transmitting light optical fiber and the receiving light optical fiber.

The invention of claim 23 is the probe of claim 22, wherein the optical fiber composing the illuminating section is latched to a cutout section formed on an outer circumference of the holding member.

The invention of claim 24 is the probe of claim 23, wherein the cutout section is formed in a tapered shape.

The invention of claim 25 is the probe of claim 22, wherein the optical fiber composing the illuminating section is inserted through a through hole formed on the holding member and latched.

The invention of claim 26 is the probe of claim 9, wherein illuminating lenses which correspond to each one of the illuminating light guiding optical fiber and which diffuse illuminating light guided by the illuminating light guiding optical fiber are provided by separate independent components.

The invention of claim 27 is the probe of claim 26, further comprising a light guiding lens which guides the excitation light and the emitted light to a tip end side of the transmitting light optical fiber and the receiving light optical fiber, wherein a flange section is formed outside the effective diameter of the light guiding lens, and the illuminating light guiding optical fiber is inserted through a through hole or groove formed in the flange section and latched.

The invention of claim 28 is the probe of claim 26, wherein the illuminating lens is composed from a plurality of ball lenses with respect to one of the illuminating light guiding optical fiber.

The invention of claim 29 is the probe of any one of claims 1 to 7, further comprising an illuminating light guiding optical fiber which guides illuminating light from an illuminating light source to a probe tip end section as the illuminating section and an antifouling hood provided at the probe tip end, wherein a tip end section of the illuminating light guiding optical fiber extending in a tip end direction than the transmitting light optical fiber and the receiving light optical fiber is held by the antifouling hood.

The invention of claim 30 is a diagnosis device comprising:
a probe described in any one of claims 1 to 29;
a base unit connected to a rear end of the probe; and
an operation input device connected to the base unit,
wherein the base unit controls turning on and off of the illuminating section based on an operation signal from the operation input device.

The invention of claim 31 is the diagnosis device of claim 30, wherein the base unit is provided with a spectroscopic analysis section which performs spectroscopic analysis of light taken in from the receiving light optical fiber and the analysis target of the spectroscopic analysis section is light taken in from the receiving light optical fiber when the illuminating section is turned off.

The invention of claim 32 is the diagnosis device of claim 31, wherein the base unit is provided with an interface which inputs an image signal of an endoscope and an image composite section which overlaps and combines an image of the endoscope and an analysis result by the spectroscopic analysis section.

The invention of claim 33 is the diagnosis device of claim 30, wherein the base unit includes an interface which inputs an image signal of an endoscope and a light modulating section which modulates light of the illuminating section based on an image signal of the endoscope.

The invention of claim 34 is a method for using a diagnosis device of any one of claims 30 to 33 wherein the probe is inserted through the endoscope channel of the endoscope main body and after the illumination of the endoscope main body is turned off, the illuminating section is used as illumination when imaging is performed by the imaging section of the endoscope main body.

### Advantageous Effect of the Invention

According to the present invention, imaging by the imaging section of the endoscope main body can be performed while illuminating the observation target region with the illuminating section included in the probe and a probe with preferable observation properties can be provided. Specifically, when illumination is provided on the endoscope main body, by turning off the illumination and turning on and off the illuminating section included in the probe, it is possible to switch between an environment suitable for imaging by an endoscope main body and an environment suitable for observation by the probe. Therefore, it is possible to obtain the effect of performing observation in which both preferable operability and observation properties are achieved and being able to use a general endoscope main body including an endoscope channel.

### Brief Description of the Drawings

FIG. 1 is a diagram showing an entire configuration of the endoscope system according to an embodiment of the present invention;
FIG. 2 is a perspective diagram showing an endoscope according to an embodiment of the present invention;
FIG. 3A is a cross sectional schematic diagram of a tip end section of a probe according to an embodiment of the present invention;
FIG. 3B is a cross sectional schematic diagram of a tip end section of a probe according to an embodiment of the present invention;
FIG. 3C is a cross sectional schematic diagram of a tip end section of a probe according to an embodiment of the present invention;
FIG. 4A is a cross sectional schematic diagram of a probe according to an embodiment of the present invention;
FIG. 4B is a cross sectional schematic diagram of a probe according to an embodiment of the present invention;
FIG. 5A is a schematic diagram of a main configuration of a tip end section of a probe according to an embodiment of the present invention;
FIG. 5B is a schematic diagram of a main configuration of a tip end section of a probe according to an embodiment of the present invention;
FIG. 6 is a schematic diagram of a main configuration of a tip end section of a probe according to an embodiment of the present invention;
FIG. 7A is a schematic diagram of a main configuration of a tip end section of a probe according to an embodiment of the present invention;
FIG. 7B is a schematic diagram of a main configuration of a tip end section of a probe according to an embodiment of the present invention;
FIG. 8A is a schematic diagram of a main configuration of a tip end section of a probe according to an embodiment of the present invention;
FIG. 8B is a schematic diagram of a main configuration of a tip end section of a probe according to an embodiment of the present invention;
FIG. 9 is a schematic diagram of a main configuration of a tip end section of a probe according to an embodiment of the present invention;
FIG. 10 is a schematic diagram of a main configuration of a tip end section of a probe according to an embodiment of the present invention;
FIG. 11A is a cross sectional diagram of a light guiding lens according to an embodiment of the present invention;
FIG. 11B is a perspective diagram of a light guiding lens according to the embodiment;
FIG. 12A is a cross sectional diagram of a light guiding lens according to an embodiment of the present invention;
FIG. 12B is a perspective diagram b of a light guiding lens according to the embodiment;
FIG. 13A is a schematic diagram of a main configuration of a tip end section of a probe according to an embodiment of the present invention;
FIG. 13B is a schematic diagram of a main configuration of a tip end section of a probe according to an embodiment of the present invention;
FIG. 14 is a schematic diagram of a main configuration of a tip end section of a probe according to an embodiment of the present invention;
FIG. 15 is a schematic diagram of a main configuration of a tip end section of a probe according to an embodiment of the present invention;
FIG. 16 is a transparent perspective diagram of a tip end section of a probe according to an embodiment of the present invention;
FIG. 17A is a transparent perspective diagram of a tip end section of a probe according to an embodiment of the present invention;
FIG. 17B is a cross sectional diagram of a tip end section of a probe according to the embodiment;
FIG. 18 is a transparent perspective diagram of a tip end section of a probe according to an embodiment of the present invention;
FIG. 19 is a perspective diagram of a tip end section of a probe according to an embodiment of the present invention;
FIG. 20A is a side view diagram of a tip end section of an optical fiber according to an embodiment of the present invention;
FIG. 20B is a side view diagram of a tip end section of an optical fiber according to an embodiment of the present invention;
FIG. 21 is a perspective diagram of a tip end section of an optical fiber and a small lens according to an embodiment of the present invention;
FIG. 22A is a perspective diagram of a tip end section of an optical fiber according to an embodiment of the present invention;
FIG. 22B is a perspective diagram of a tip end section of an optical fiber according to an embodiment of the present invention;
FIG. 23 is a cross sectional diagram of a tip end section of a probe according to an embodiment of the present invention;
FIG. 24 is a perspective diagram of a plane convex lens according to an embodiment of the present invention;
FIG. 25 is a perspective diagram of a cylinder shaped member in which a plane convex lens is fixed according to an embodiment of the present invention;
FIG. 26 is a perspective diagram of a cylinder shaped member in which a plane convex lens is provided according to an embodiment of the present invention; and
FIG. 27 is a perspective diagram of a cylinder shaped member, in which a plane convex lens is provided, is filled with adhesive according to an embodiment of the present invention.

### Embodiment for Carrying Out the Invention

An embodiment of the present invention is described below with reference to the drawings. The following is an embodiment of the present invention and does not limit the present invention.

### (Example of Entire Configuration)

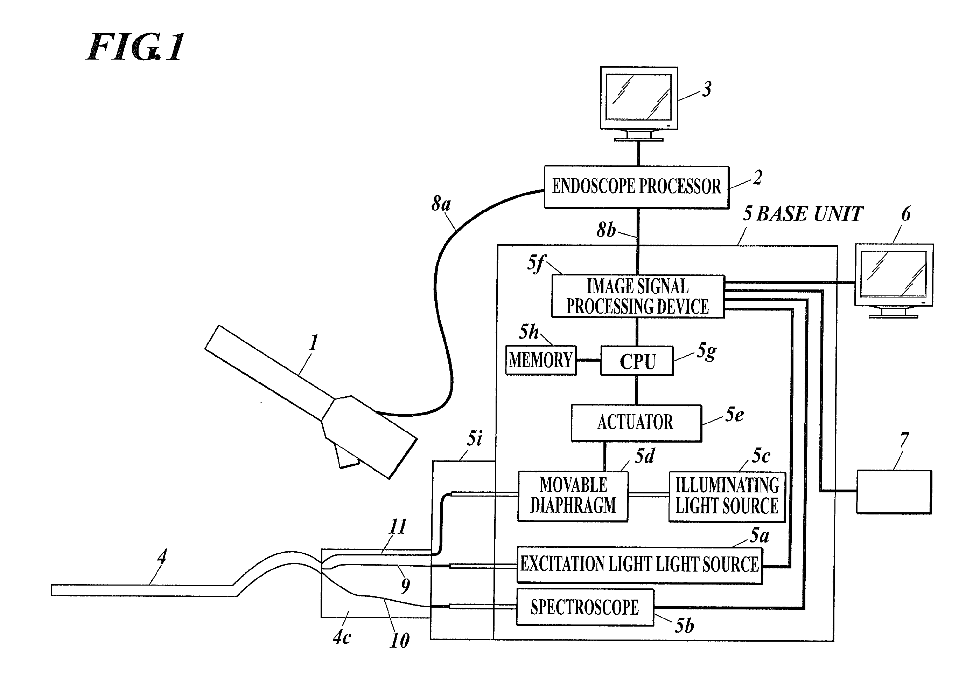

The endoscope system of the present embodiment is a fluorescence diagnosis device added to an endoscope.
As shown in FIG. 1, the endoscope system of the present embodiment is configured including an endoscope main body 1, an endoscope processor 2, an endoscope display monitor 3, a probe 4, a base unit 5 of the probe 4, a display monitor 6 which displays an output image of the base unit 5, and an operation input device 7 connected to the base unit 5.
An electronic camera and illumination is provided on a tip end of the endoscope main body 1. An image signal cable 8a which sends out an image signal imaged by the electronic camera is connected to the endoscope processor 2, and the endoscope processor 2 outputs the image to the endoscope display monitor 3. The on and off of the illumination of the endoscope main body 1 can be controlled by operating the operating panel of the endoscope processor 2. The image signal is input to the base unit 5 from the endoscope processor 2 by the image signal cable 8b.

Inside the probe 4, a transmitting light optical fiber 9, a receiving light optical fiber 10, and an illuminating light guiding optical fiber 11 are passed through the probe 4 in the longitudinal direction and the above reach the tip end section of the probe 4.

The base unit 5 includes an excitation light light source 5a, a spectroscope 5b, an illuminating light source 5c, a movable diaphragm 5d, an actuator 5e of the movable diaphragm 5d, an image signal processing device 5f, a CPU (Central Processing Unit) 5g, a storage device 5h and a connector 5i. The image signal processing device 5f includes an input/output interface. A connector 4c configured in the rear end of the probe 4 can be attached and detached freely to the connector 5i.

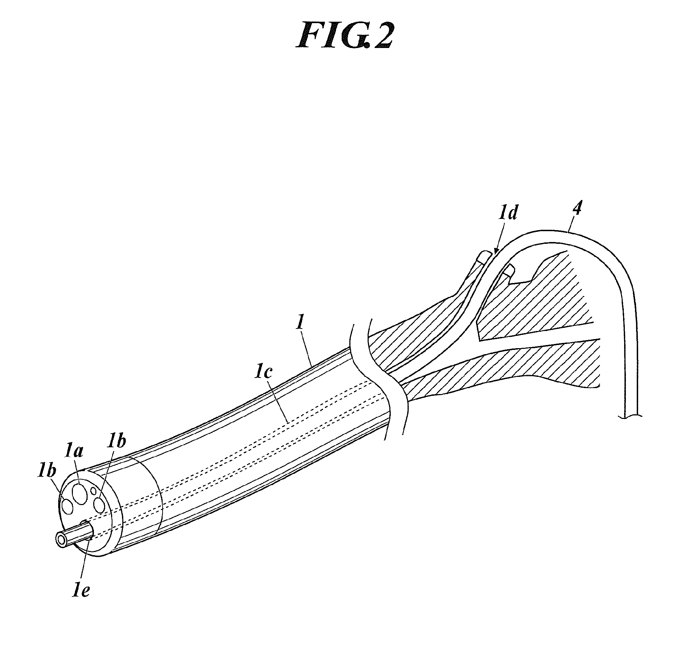

As shown in FIG. 2, an electronic camera 1a and illumination 1b is provided at the tip end of the endoscope main body 1. As shown in the diagram, an endoscope channel 1c is formed in the endoscope main body 1. The endoscope channel 1c is formed to communicate from an inserting opening 1d, which is provided branching out from the main stem of the endoscope main body 1, to a tip end opening 1e, which opens to the tip end face of the endoscope main body 1.
The probe 4 is inserted from the inserting opening 1d and is inserted in the endoscope channel 1c to reach the tip end opening 1e. The tip end section of the probe 4 projects out from the tip end opening 1e depending on the inserted length of the probe 4. In order to determine the tip end position of the probe 4 in relation with the tip end of the endoscope main body 1 to a desired position, it is preferable to provide a mark showing the inserted length on the outer circumference of the probe 4 or to provide a stopper which latches to the inserting opening 1d.

The excitation light is guided by the transmitting light optical fiber 9 from the excitation light light source 5a, and the excitation light is irradiated on the observation target region of the biological tissue such as the esophageal inner wall or the gastric inner wall. The emitted light emitted from the observation target region due to the excitation light is received by the receiving light optical fiber 10 and guided to the spectroscope 5b. Fluorescence is generated according to the lesion state due to the excitation light of the irradiated observation target region. When fluorescence is generated, the light returning from the observation target region including the fluorescence enters the receiving light optical fiber 10. The light guided by the receiving light optical fiber 10 is input to the spectroscope 5b of the base unit 5. The fluorescence is, in a broad meaning, when irradiated material is irradiated by X-ray, ultraviolet ray, and visible light ray, the electron is excited by absorbing the energy and the excess energy when returning to the ground state is discharged as electromagnetic ray. Here, due to the excitation light, fluorescence with a wavelength different from the wavelength of the excitation light is generated as the returning light. Therefore, the fluorescence is detected, the light is guided by the spectroscope 5b of the base unit 5 through the receiving light optical fiber 10, and the lesion state of the detection target is detected by analyzing spectral distribution. The information of spectral distribution is input from the spectroscope 5b to the CPU 5g.

The illuminating light guiding optical fiber 11 guides illuminating light from the illuminating light source 5c which passes through the movable diaphragm 5d to the tip end section of the probe 4.
The illuminating light guided by the illuminating light guiding optical fiber 11 is irradiated to the tip end direction of the endoscope main body 1 and is used to image the observation target region with the electronic camera 1a. According to a configuration which guides illuminating light from a light source by an optical fiber, an illuminating section can be attached to the probe without making the probe large. Moreover, the light source can be separated from the affected portion, and therefore it is highly safe to the living body.
The CPU 5g controls the actuator 5e based on the image signal of the endoscope input through the image signal processing device 5f and adjusts the opening of the movable diaphragm 5d to modulate the light of the illumination to an amount of light suitable for imaging.

The image of the endoscope is displayed as output on the display monitor 6 based on the image signal of the endoscope input through the image signal processing device 5f.
Based on the image signal of the endoscope input through the image signal processing device 5f and the spectral distribution information, the CPU 5g generates a composite image in which the image of the endoscope and the fluorescence analysis result are overlapped on each other and the composite image is output to the display monitor 6. The storage device 5h includes a RAM used as a work memory for the CPU 5g and a hard disk and SSD to accumulate data.

The operation input device 7 includes a foot switch. The foot switch is operated so that the CPU 5g of the base unit 5 controls the illuminating light source 5c or the movable diaphragm 5d based on the operation signal from the operation input device 7 to control the on and off of the illumination. The operator (diagnoser) often holds the endoscope with both hands to insert and operate the endoscope, and there is not much allowance to use the hands for fluorescence diagnosis. Therefore, the foot switch is useful.
The fluorescence diagnosis target of the CPU 5g is light taken in from the receiving light optical fiber 10 when the illumination is turned off as described above and the narrowing of the fluorescence analysis target by the CPU 5g is performed based on the operation signal from the operation input device 7.

### (Steps of Use)

The following is a description according to the steps of use when fluorescence diagnosis is performed using the endoscope system.
First, the light source of the illumination 1b included in the endoscope main body 1 is turned on, the endoscope main body 1 is inserted in the body and when the tip end section (imaging camera mounting section) reaches the position where fluorescence diagnosis is desired, the probe 4 is inserted through the endoscope channel 1c to reach the above position.
Next, the light source of the illumination 1b included in the endoscope main body 1 is turned off. This is because if the illumination is kept on, the illumination becomes a disturbance to the fluorescence diagnosis. When the illumination 1b is turned off, the endoscope image is blacked out and nothing is shown on both the endoscope display monitor 3 and the display monitor 6.
Here, the illuminating light source 5c is turned on with the foot switch of the operation input device 7 to illuminate the affected portion from the tip end of the probe 4.
Then, the image for the endoscope display monitor 3 and the display monitor 6 is obtained again, and the operator can perform operation similarly to a normal endoscope by looking at the endoscope image through the endoscope display monitor 3 or the display monitor 6.
The region on which fluorescence diagnosis is performed in the present embodiment is a narrow region similar to a very small point compared to an image of a wide range obtained by the endoscope. Therefore, it is preferable to be able to understand which region observed by the probe 4 corresponds to which region in the endoscope image. It is preferable to be able to determine unambiguously the inserted length by providing a mark or stopper on a portion of the rear end section of the probe (side connected to the base unit) as described above. By setting relation of position between the position, direction and viewing range of the electronic camera and the position and direction of the optical system of the transmitting/receiving light of the probe to a suitable relation of position, a relation to perform fluorescence observation at a predetermined position of the endoscope image, preferably at substantially the center section of the endoscope image is configured and an environment highly useful for the operator and/or diagnoser can be provided.

Next, when the fluorescence diagnosis is started, operation by the foot switch is performed again.
Then, the CPU 5g outputs an instruction to close the movable diaphragm 5d or to turn off the power source of the illuminating light source to turn off the illumination included in the probe 4. Here, the image before turning off the illumination is stored in the storage section in the base unit 5.
Next, the optical path of the excitation light light source is opened and the excitation light is transmitted through the guiding light path of the transmitting light optical fiber 9 to be irradiated on the biological tissue (lumen). Then, the biological tissue emits fluorescence according to its state.
The fluorescence is received through the guiding light path of the receiving light optical fiber 10 and is guided to the spectroscope 5b.
The spectroscope 5b and the CPU 5g analyze the fluorescence to judge the state of the tissue.
Then, image processing to overlap the fluorescence diagnosis result with the endoscope image obtained in the base unit 5 before turning off the illumination of the probe 4 is performed, and the image is displayed on the display monitor 6 connected to the base unit 5 side.
According to the above, the image reflecting the fluorescence diagnosis result is displayed together with the endoscope image on the display monitor 6 of the base unit 5 side when fluorescence diagnosis is performed. Therefore, this becomes a very useful diagnosis tool for the operator.

### (Configuration of Probe Tip End Section Part 1)

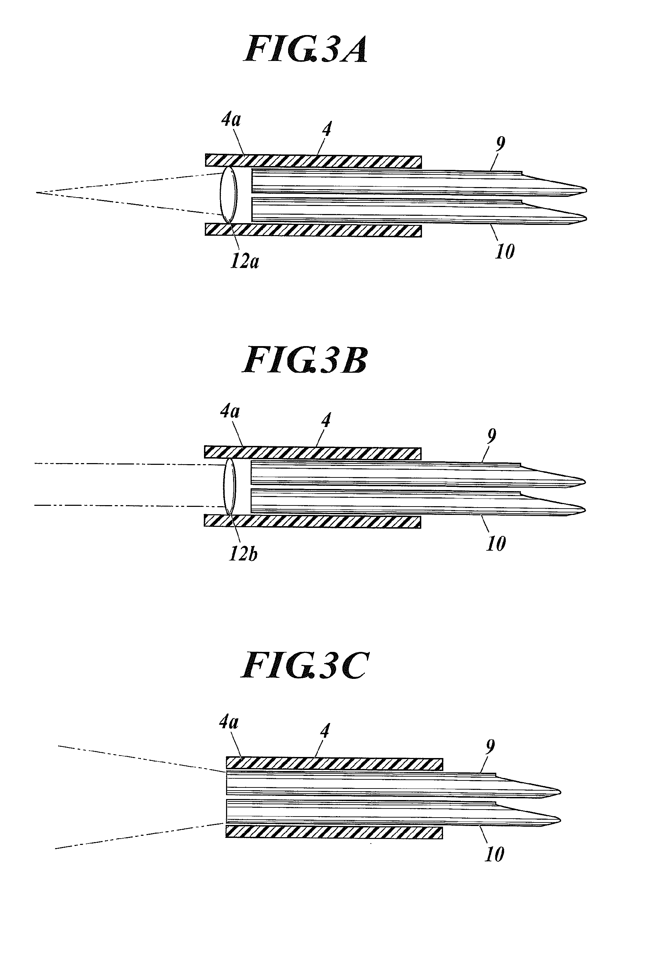

Next, the form of the tip end section of the probe 4 is described with reference to FIG. 3A, FIG. 3B and FIG. 3C.
As shown in FIG. 3A and FIG. 3B, light guiding lenses 12a and 12b are provided to guide excitation light and emitted light to the tip end side of the transmitting light optical fiber 9 and the receiving light optical fiber 10. In FIG. 3A, FIG. 3B and FIG. 3C, the optical fibers 9 and 10 are drawn exceptionally wide and the configuration of the illuminating section is not illustrated.
The light guiding lens 12a is a light condensing lens and the light guiding lens 12b is a collimated lens.
The probe 4 includes a tube 4a with water shielding properties to form the outer circumferential wall. Watertight processing such as adhesion and fusion is performed between the light guiding lenses 12a and 12b and the tube 4a so that the tip end opening of the tube 4a is a configuration with a waterproof seal by the light guiding lenses 12a and 12b. A covering member instead of the light guiding lenses 12a and 12b can be applied as a sealing member.
The configuration shown in FIG. 3C does not include the above described light guiding lens and the tip end face of the transmitting light optical fiber 9 and the receiving light optical fiber 10 is exposed to the tip end of the probe 4. In this case, the configuration between the tip end section of the transmitting light optical fiber 9 and the receiving light optical fiber 10 and the tube 4a is a waterproof seal by processing such as filling etc., with a resin material, etc.

### (Modification Example of Illuminating Section)

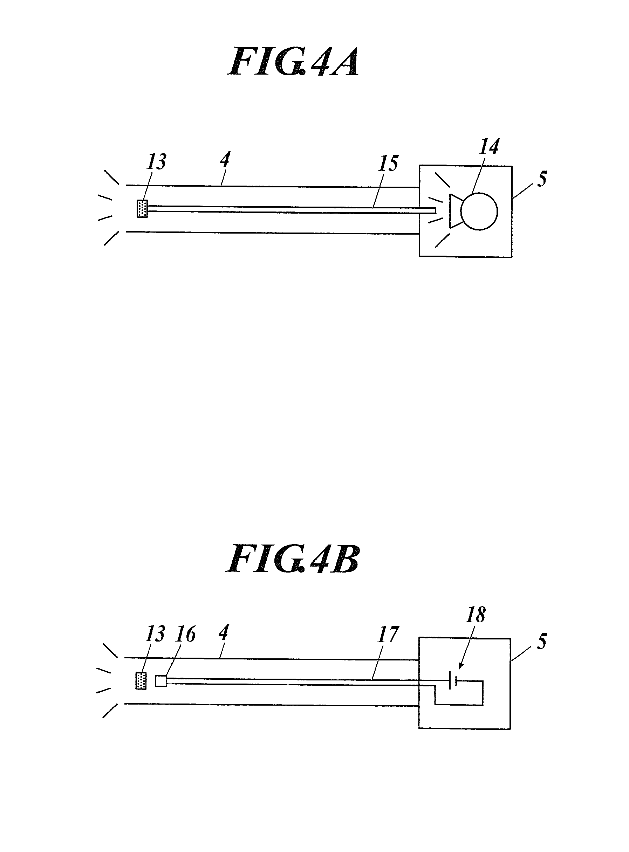

A light emitting diode can be applied instead of the above configuration as the illuminating section included in the probe 4. In this case, a light emitting diode is provided at the tip end section of the probe 4, and the power source line of the light emitting diode is passed in the longitudinal direction of the probe 4 to be guided to the base unit 5 to be connected to the power source provided in the base unit 5. When a light emitting diode is used, the size becomes larger compared to guiding light with an optical fiber. However, the size can be smaller than when using a light source such as a fluorescence tube.
A light emitting material piece which emits white color fluorescence can be applied as the illuminating section. In this case, as shown in FIG. 4A and FIG. 4B, a light emitting material piece 13 is provided on the tip end section of the probe 4. As a section to irradiate excitation light to the light emitting material piece 13 so that the light emitting material piece 13 emits light, an optical fiber 15 which guides the excitation light from the power source 14 to the light emitting material piece 13 as shown in FIG. 4A can be applied or a light emitting diode 16 provided next to the light emitting material piece 13 as shown in FIG. 4B can be applied. The power source 14 is provided in the base unit 5. The power source line 17 of the light emitting diode 16 is passed through the probe 4 in the longitudinal direction to be guided to the base unit 5 to be connected to a power source 18 provided in the base unit 5. As described above, according to a configuration which provides a light emitting material at the tip end of the probe, the size of the tip end of the probe becoming large can be avoided.

### (Configuration of Probe Tip End Section Part 2)

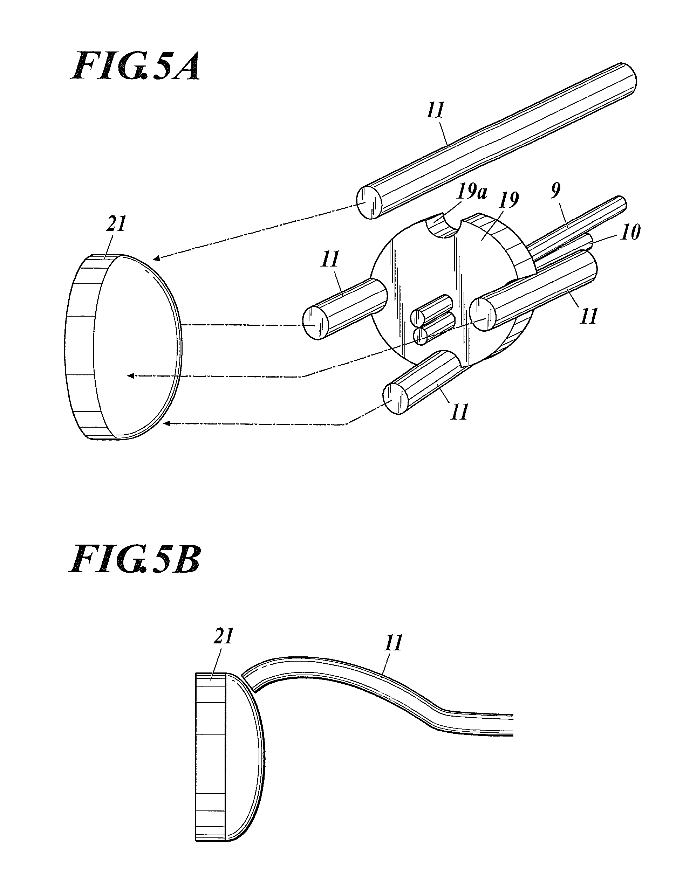

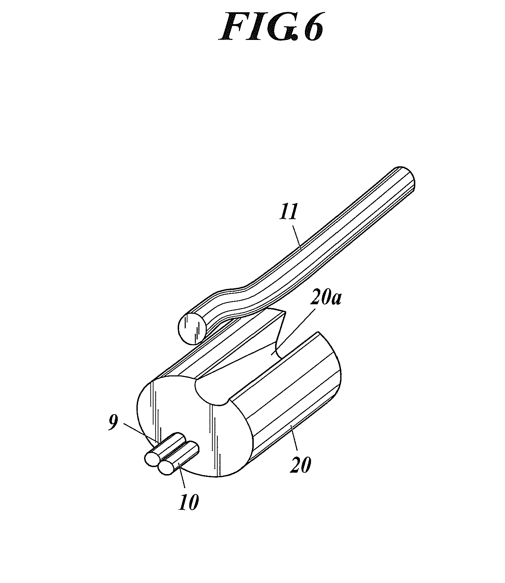

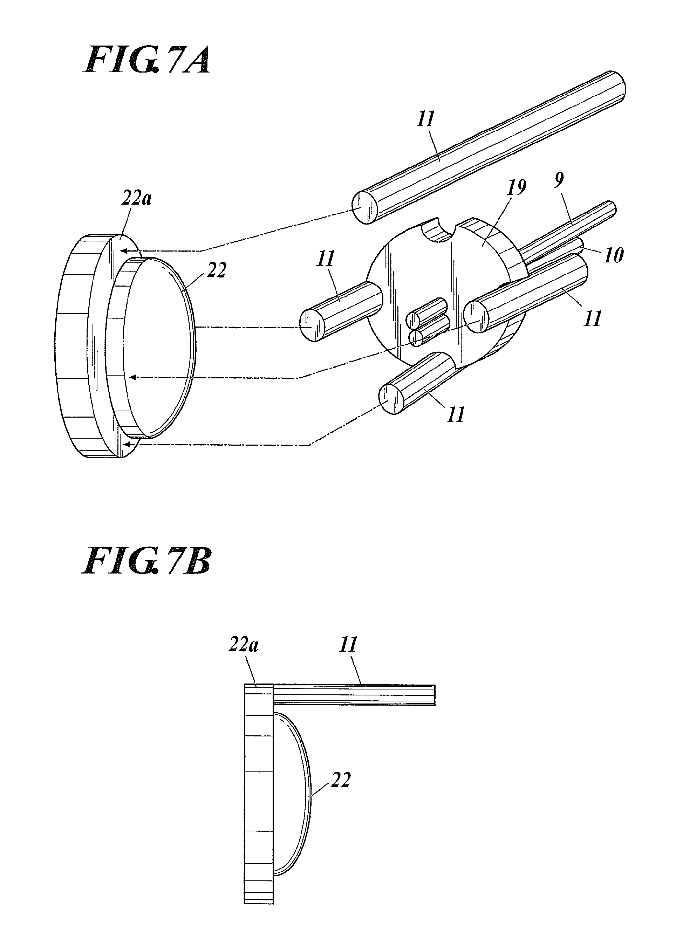

The form of providing and holding the transmitting light optical fiber 9, the receiving light optical fiber 10 and the illuminating light guiding optical fiber 11 is described.
In order to hold the above optical fibers, a holding member 19 as shown in FIG. 5A is shared. The holding member 19 can also be applied when the illuminating light guiding optical fiber 11 is the above described optical fiber 15 which guides the excitation light from the light source 14 to the light emitting material piece 13.
As shown in FIG. 5A and FIG. 5B, the illuminating light guiding optical fiber 11 is set and latched in a cutout section 19a formed on the outer circumference of the holding member 19. For the purpose of easy understanding, FIG. 5A illustrates some of the illuminating light guiding optical fibers in a state before attachment (this point can be similarly said for the later described FIG. 7A, FIG. 8A and FIG. 13B). The holding member 20 shown in FIG. 6 can be applied as the holding member. In the holding member 20, the cutout section 20a which holds the optical fiber 11 is formed in a tapered shape. FIG. 6 illustrates an example in which only one cutout section 20a is provided, however the necessary number can be provided.
The transmitting light optical fiber 9 and the receiving light optical fiber 10 are inserted through the through hole formed in the holding members 19 and 20 and latched.
The optical fibers 11 and 15 can also be inserted through the through hole formed in the holding member and latched.

In the configuration shown in FIG. 5A and FIG. 5B, the illuminating light guiding optical fiber 11 is provided so that the output light end is in contact toward the outer circumferential section of a light guiding lens 21 in order to allow the guided illuminating light to enter the outer circumferential section of the light guiding lens 21. The form of fixing the optical fiber with the lens is schematically shown in FIG. 5B (FIG. 7B, FIG. 8B, FIG. 9 and FIG. 10 similarly describe a schematic diagram). Adhesive, etc. is suitably used for fixing.

In the configuration shown in FIG. 7A and FIG. 7B, a flange section 22a is formed outside the effective diameter of the light guiding lens 22. The illuminating light guiding optical fiber 11 is provided so that the output light end faces the flange section 22a in order to allow the guided illuminating light to enter the flange section 22a. A diffusing section to diffuse the illuminating light can be formed in the flange section 22a and the output light end of the illuminating light guiding optical fiber 11 can be in contact with the flange section 22a or a diffusing plate to diffuse illuminating light can be provided sandwiched between the flange section 22a and the output light end of the illuminating light guiding optical fiber 11 to be in contact with each other. Adhesive, etc. is suitably used for fixing.

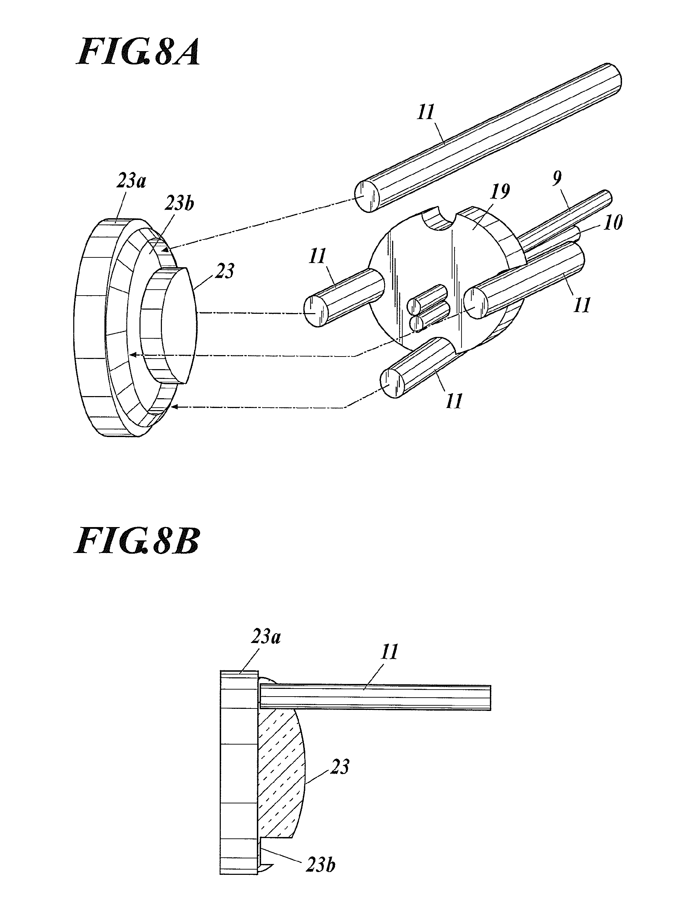

In the configuration shown in FIG. 8A and FIG. 8B, a flange section 23a is formed outside the effective diameter of a light guiding lens 23. A circumferential groove 23b in a circular shape is formed in the flange section 23a. The output light end section of the illuminating light guiding optical fiber 11 is provided inserted in the circumferential groove 23b. Adhesive, etc. is suitably used for fixing.

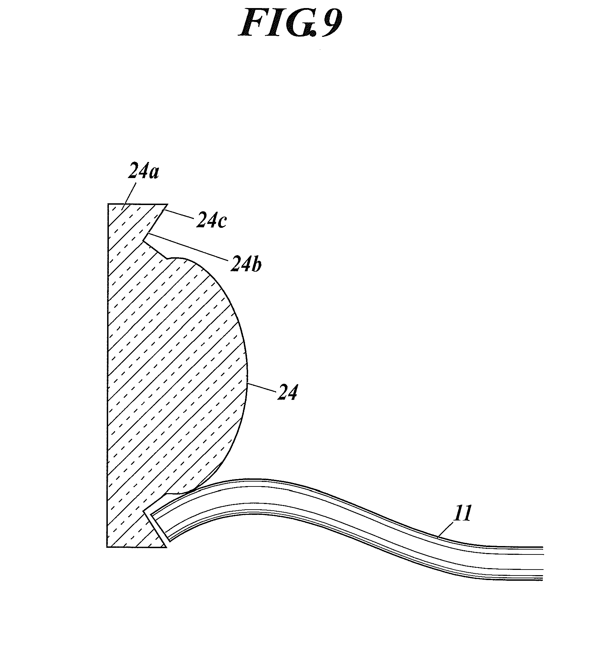

In the configuration shown in FIG. 9, a light guiding lens 24 is applied. A flange section 24a is formed outside the effective diameter of the light guiding lens 24. A groove 24b is formed in the flange section 24a. An inclined face 24c is formed as the outside face of the groove 24b. The output light end of the illuminating light optical fiber 11 is provided against the inclined face 24c. With this, the output light end of the illuminating light guiding optical fiber 11 is provided inclined outward. The facing face of the flange section 24a facing the output light end of the illuminating light guiding optical fiber 11 is the inclined face 24c and the inclined face 24c is inclined so that the normal direction is inward.

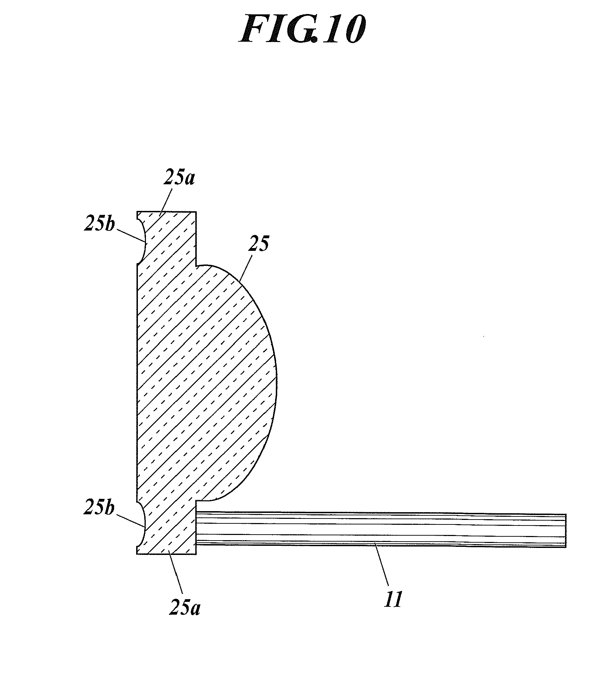

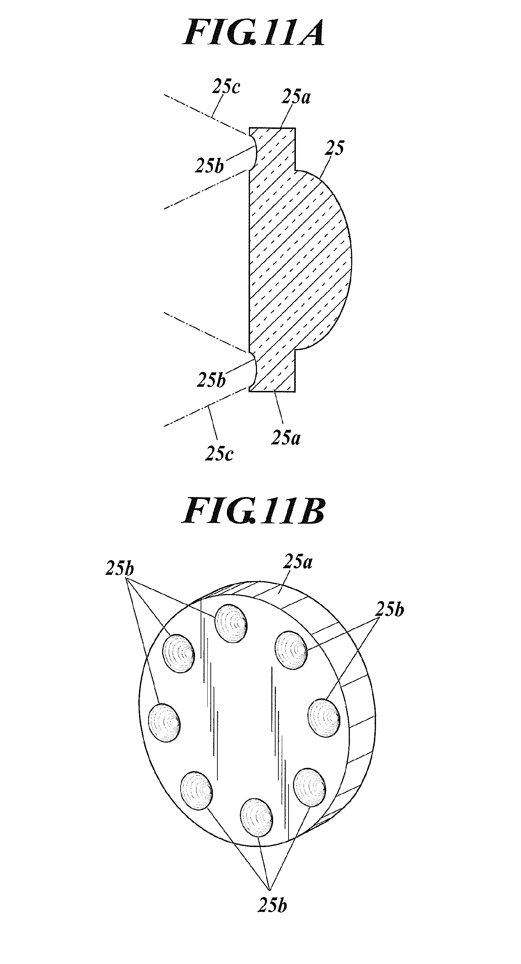

In the configuration shown in FIG. 10, FIG. 11A and FIG. 11B, a light guiding lens 25 is applied. A flange section 25a is formed outside the effective diameter of the light guiding lens 25. An illuminating lens 25b is formed in the flange section 25a. The illuminating lens 25b is provided corresponding to each one of the illuminating light guiding optical fiber 11. The output light end of the illuminating light guiding optical fiber 11 is in contact with the flange section 25a. The illuminating lens 25b is formed with a concave face, and the irradiating angle of the irradiating light which enters the flange section 25a from the illuminating light guiding optical fiber 11 is enlarged with the illuminating lens 25b. The illuminating light which exits from the illuminating lens 25b in FIG. 11A is shown as 25c.
As shown in FIG. 11A and FIG. 11B, a plurality of illuminating lenses 25b are provided to correspond to a plurality of illuminating light guiding optical fibers 11. The optical axes of the plurality of illuminating lenses 25b are provided on a same concentric circle.

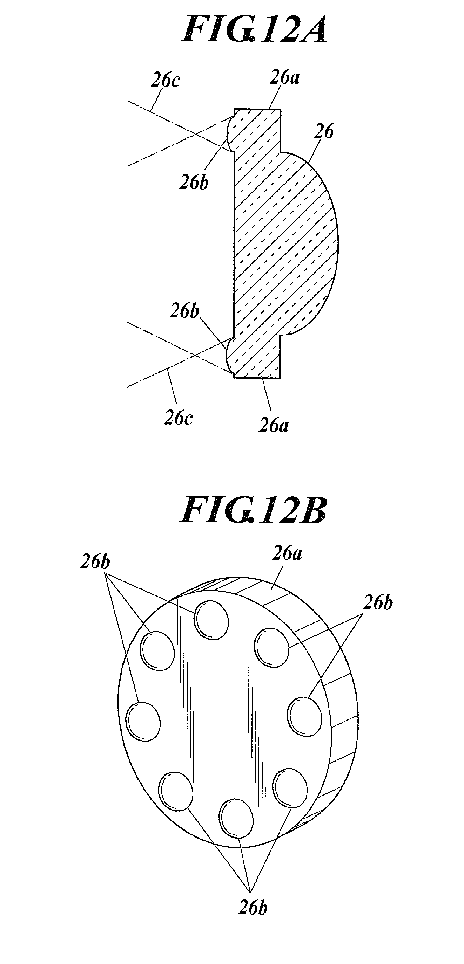

Instead of the light guiding lens 25 shown in FIG. 11A and FIG. 11B, a light guiding lens 26 shown in FIG. 12A and FIG. 12B can be applied. A flange section 26a is formed outside the effective diameter of the light guiding lens 26. An illuminating lens 26b is formed in the flange section 26a. The illuminating lens 26b is provided corresponding to each one of the illuminating light guiding optical fibers 11. Similar to FIG. 10, the output light end of the illuminating light guiding optical fiber 11 is in contact with the flange section 25a. The illuminating lens 26b is formed with a convex face, and the illuminating angle is enlarged after the illuminating light which enters the flange section 25a from the illuminating light guiding optical fiber 11 is condensed. The illuminating light which exits from the illuminating lens 26b in FIG. 12A is shown as 26c.

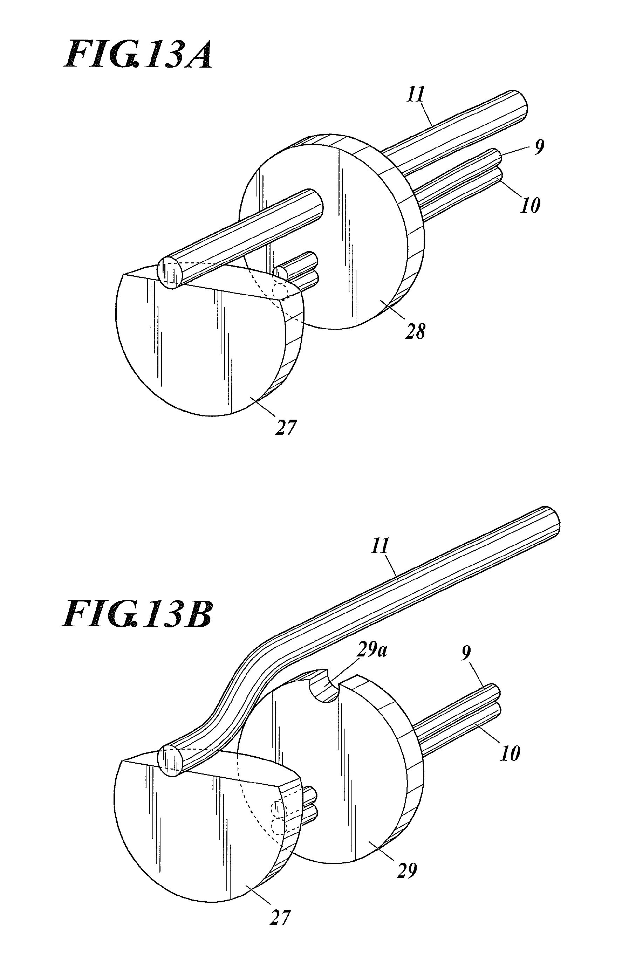

In the configuration shown in FIG. 13A and FIG. 13B, a light guiding lens 27 is applied. The outer shape of the light guiding lens 27 is formed in a D shape in which a portion of a round shape is missing. An electric line and optical fiber which compose the illuminating section is passed in a space corresponding to the missing section. FIG. 13A and FIG. 13B show a configuration in which an illuminating light guiding optical fiber 11 is passed. Holding members 28 and 29 which hold optical fibers 9, 10 and 11 are applied. The holding member 28 shown in FIG. 13A is a form where the optical fibers 9, 10 and 11 pass through to be held. The holding member 29 shown in FIG. 13B is a form where the optical fibers 9 and 10 pass through to be held, and the optical fiber 11 is set and held in a cutout 29a formed in the outer circumferential section. When a light emitting diode is provided in the tip end section of the probe 4 instead of the illuminating light guiding optical fiber 11, the electric line can be passed through a space corresponding to the missing section of the D shaped light guiding lens 27.

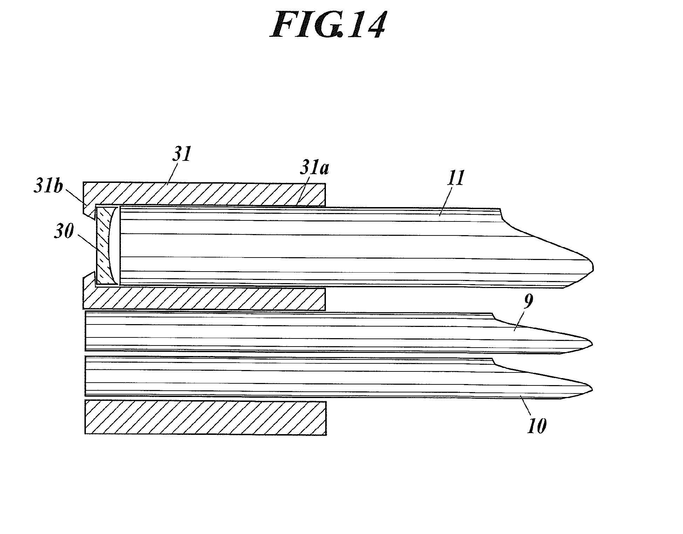

Separate independent illuminating lenses can be applied. An illuminating lens 30 as shown in FIG. 14 can be applied. The illuminating lens 30 corresponds to each one of the illuminating light guiding optical fiber 11. The illuminating lens 30 is a lens which diffuses illuminating light guided by the illuminating light guiding optical fiber 11. The illuminating lens 30 is provided with separate independent components. In other words, the illuminating lens 30 is not formed as one portion of the light guiding lens which guides the excitation light and the returning light, and when a plurality of illuminating lenses 30 are provided, the illuminating lenses 30 are provided with components different from each other. A holding member 31 is applied to fix the illuminating light guiding optical fiber 11 with the illuminating lens 30. The holding member 31 is made from zirconia, and a latching section 31b is formed to latch the front end circumferential border of the illuminating lens 30 to the inner side of the front end of an inserting hole 31a for the optical fiber 11. For example, after the illuminating lens 30 is inserted in the inserting hole 31a, an adhesive is filled in the inserting hole 31a or applied to the tip end of the illuminating light guiding optical fiber 11, and by inserting the illuminating light guiding optical fiber 11 in the inserting hole 31a, the illuminating light guiding optical fiber 11, the illuminating lens 30 and the holding member 31 are fixed with adhesive to each other and assembled.

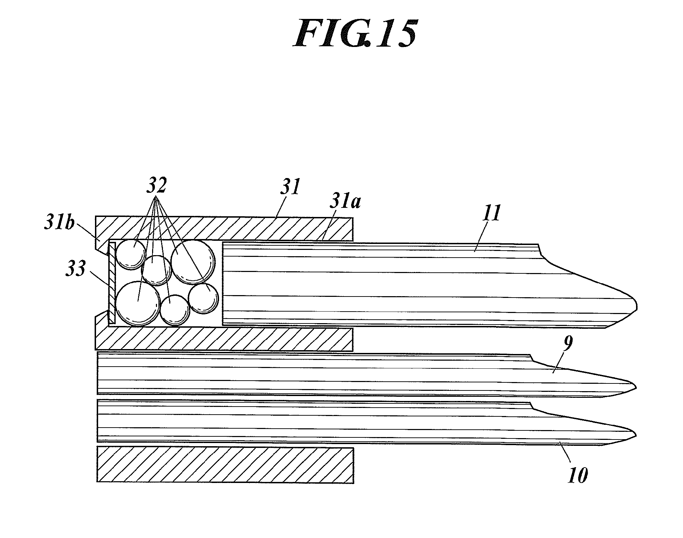

A ball lens 32 shown in FIG. 15 can be applied instead of the illuminating lens 30 shown in FIG. 14. The illuminating lens is composed of a plurality of ball lenses for each one of the illuminating light guiding optical fibers 11. A ball lens 32 with a different size can be used.
For example, after providing a cap member 33 in the inserting hole 31a of the holding member 31 to prevent the ball lens from falling out, the ball lens 32 and adhesive is filled in the inserting hole 31a of the holding member 31, and then by inserting the illuminating light guiding optical fiber 11 in the inserting hole 31a, the illuminating light guiding optical fiber 11, the ball lens 32 and the holding member 31 are fixed with adhesive to each other and assembled.
Even if a configuration shown in FIG. 14 and FIG. 15 is employed, a light guiding lens for the transmitting light optical fiber 9 and the receiving light optical fiber 10 can be provided, and the transmitting light optical fiber 9 and the receiving light optical fiber 10 can be projected out to the tip end.

### (Configuration of Probe Tip End Section Part 3)

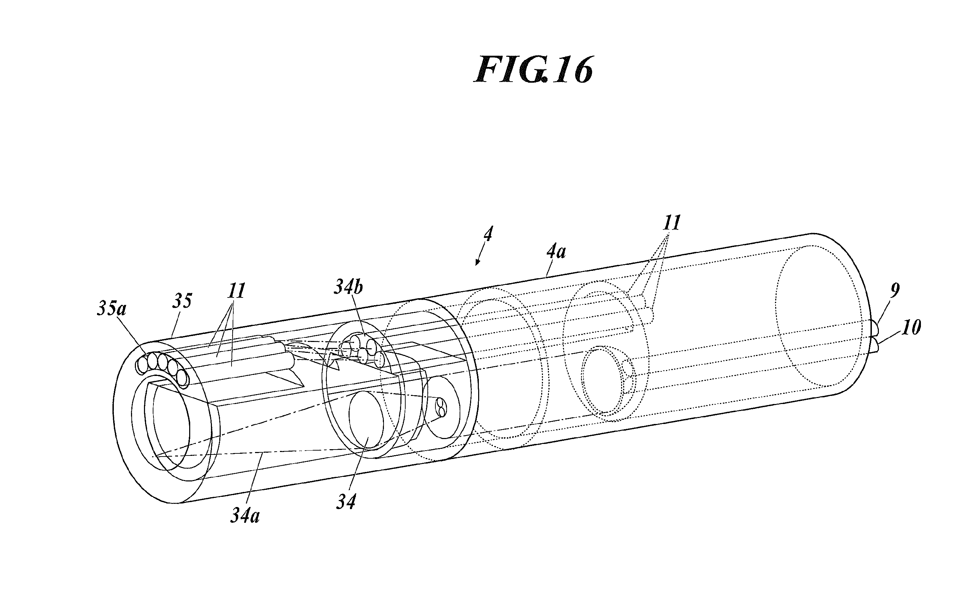

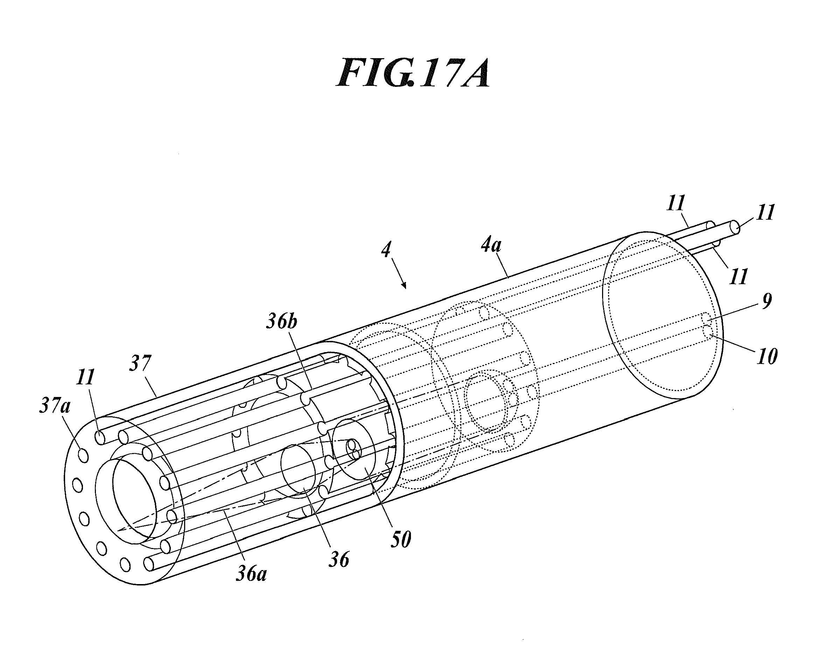

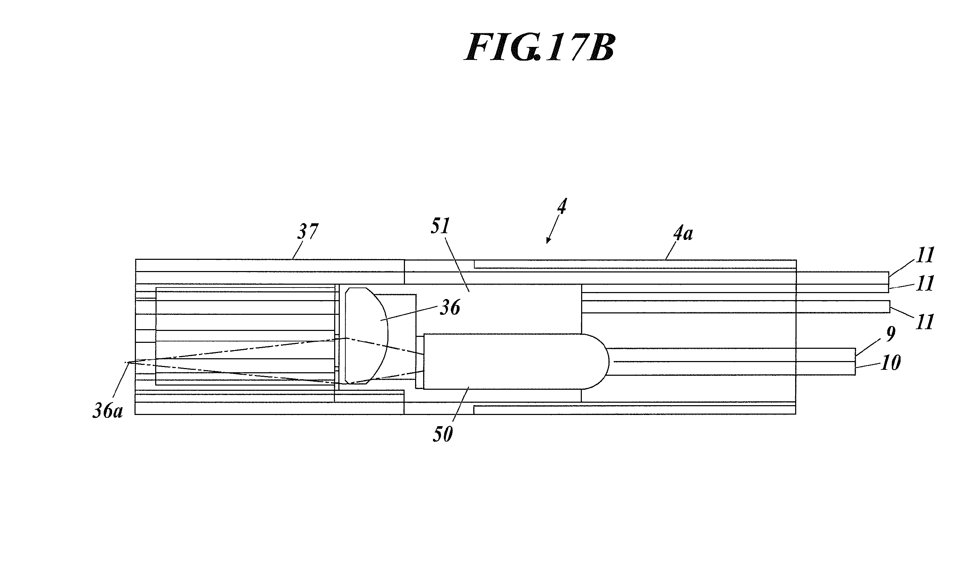

According to the configuration shown in FIG. 16, the probe 4 includes an antifouling hood 35 attached at the tip end of the probe 4 and a light guiding lens 34. As shown in FIG. 16, the light guiding lens 34 is provided at the tip end side of the transmitting light optical fiber 9 and the receiving light optical fiber 10. The excitation light 34a which exits from the transmitting light optical fiber 9 permeates the antifouling hood 35 and focuses at the tip end face of the antifouling hood 35 or on the tip end side than the tip end face of the antifouling hood 35.
The entire light guiding lens 34 including the flange section outside the effective diameter is provided in the rear end of the antifouling hood 35, and the rear end of the antifouling hood 35 and the tip end of the tube 4a are joined and sealed to be prevented.
The light guiding lens 34 is a D shape including a missing section from a round shape. The illuminating light guiding optical fibers 11, 11 and so on are inserted through a space 34b corresponding to the missing section of the light guiding lens 34 and the tip end section extending out toward the tip end direction from the transmitting light optical fiber 9 and the receiving light optical fiber 10 is inserted and held in a holding hole 35a formed in the antifouling hood 35. A groove 36b as shown in FIG. 17A can be applied instead of the space 34b. According to the configuration shown in FIG. 17A and FIG. 17B, a light guiding lens 36 and antifouling hood 37 are applied. In the antifouling hood 35 shown in FIG. 16, the holding holes 35a which hold the tip end section of the illuminating light guiding optical fibers 11, 11, and so on are concentrated on one side. However, in the antifouling hood 37 shown in FIG. 17A and FIG. 17B, the holding holes 37a which separately hold the illuminating light guiding optical fiber 11 are provided in a substantially equal interval on the circumference. In accordance with the above, the grooves 36b, 36b and so on of the flange section of the light guiding lens 36 are also provided in a substantially equal interval on the circumference. Needless to say, the groove 36b can be changed to a through hole.

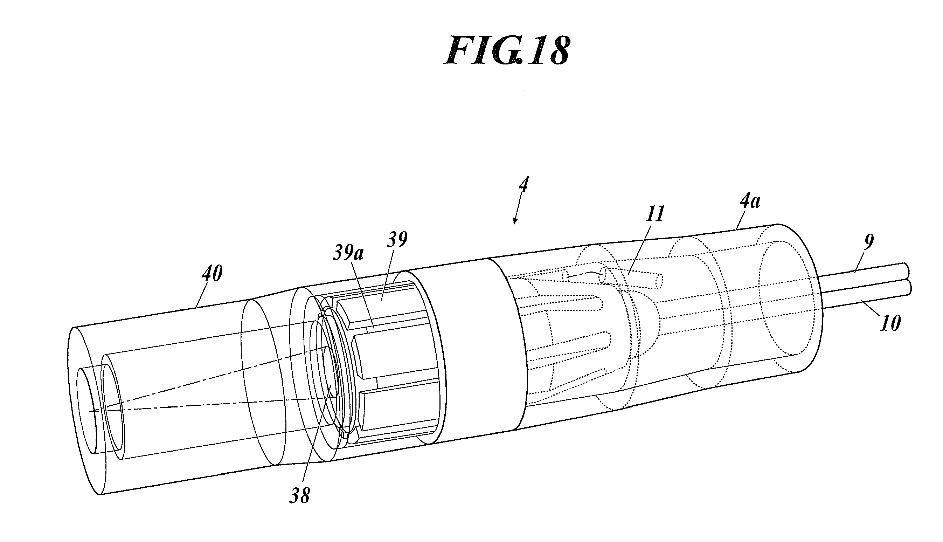

According to the configuration shown in FIG. 16, FIG. 17A and FIG. 17B, the transmitting light optical fiber 9, the receiving light optical fiber 10 and the light guiding lens on the tip end side are provided displaced from the center axis of the probe 4, however the above can be provided in the center as shown in FIG. 18.
A light guiding lens 38, a holding member 39 and an antifouling hood 40 are applied to the configuration shown in FIG. 18. Each illuminating light guiding optical fiber 11 is set and held in the groove 39a provided in the outer circumferential section in the holding member 39.

### (Application of Multi-lumen Tube)

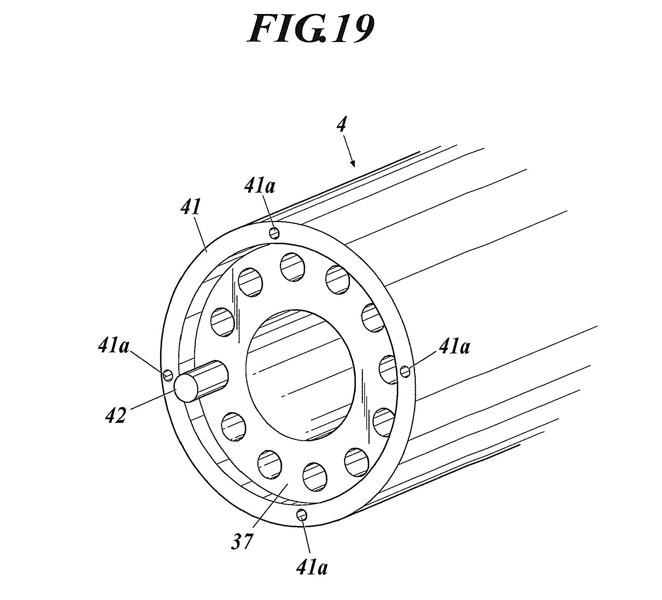

As shown in FIG. 19, a multi-lumen tube 41 can be applied as the most outer cover of the probe 4. FIG. 19 shows an example combined with the antifouling hood 37. However, the combination between the multi-lumen tube 41 and other configuration is not limited to the above and any combination is possible.
A hole 41a with water shielding properties and in communication toward the longitudinal direction is formed in the multi-lumen tube 41. The tip end opening of the hole 41a is open toward the tip end direction of the probe. A receiving opening (not shown) of the liquid injector is provided in communication with the rear end opening of the hole 41a. An injector filled with marking liquid is inserted in the receiving opening and the marking liquid can be ejected out from the tip end opening of the hole 41a. A biocompatible dye is used as a marking liquid.
Therefore, the target portion such as the esophageal inner wall or gastric inner wall can be marked according to the diagnosis result. Then after the probe 4 is removed, forceps are inserted in the endoscope channel 1, and it is possible to extract tissue to perform biological test or treatment such as excision may be possible.
As a method of marking, a biological body can be cauterized finely by a laser light emitted from an optical fiber 42. As a providing path of the optical fiber 42, the above described providing path of the illuminating light guiding optical fiber 11 can be used or the hole 41a of the multi-lumen tube 41 can be used.

### (Other Forms such as Optical Fiber)

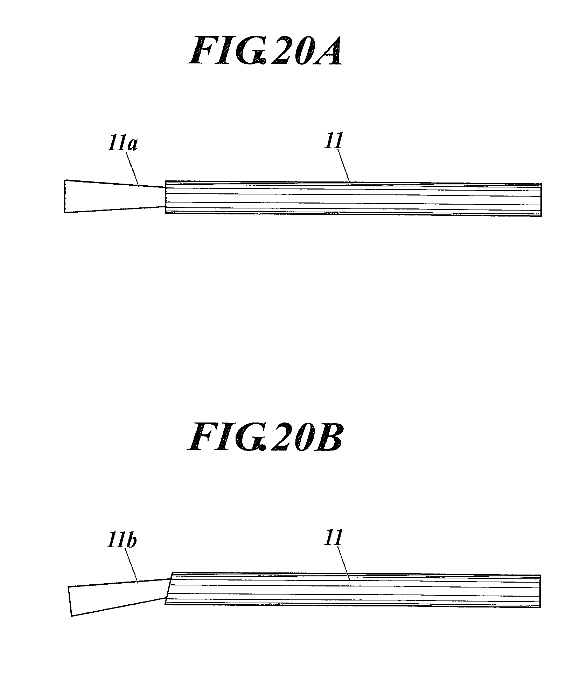

The exit end face of the illuminating light guiding optical fiber 11 can be formed perpendicular to the axis direction as shown in FIG. 20A. However, the exit end face of the illuminating light guiding optical fiber 11 can be formed diagonal to the axis direction as shown in FIG. 20B. In the optical fiber 11 shown in FIG. 20A, the exiting light 11a advances in the same axis as the optical fiber 11. However, in the optical fiber 11 shown in FIG. 20B, the exiting light 11b advances diagonally with respect to the axis of the optical fiber 11. With this, the exiting light from the optical fiber 11 can be oriented and a preferable illuminating range can be formed.

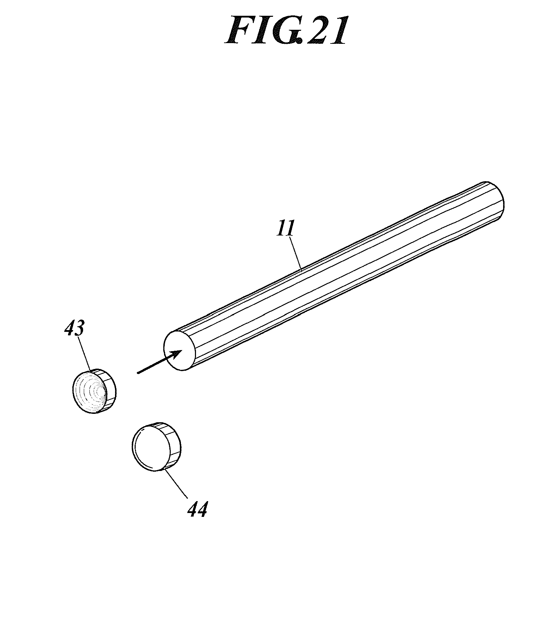

It is useful to fix with adhesive, etc. various lenses such as a small concave lens 43 or convex lens 44 to the exit end face of the illuminating light guiding optical fiber 11 as shown in FIG. 21. With this, a preferable illuminating range can be formed.

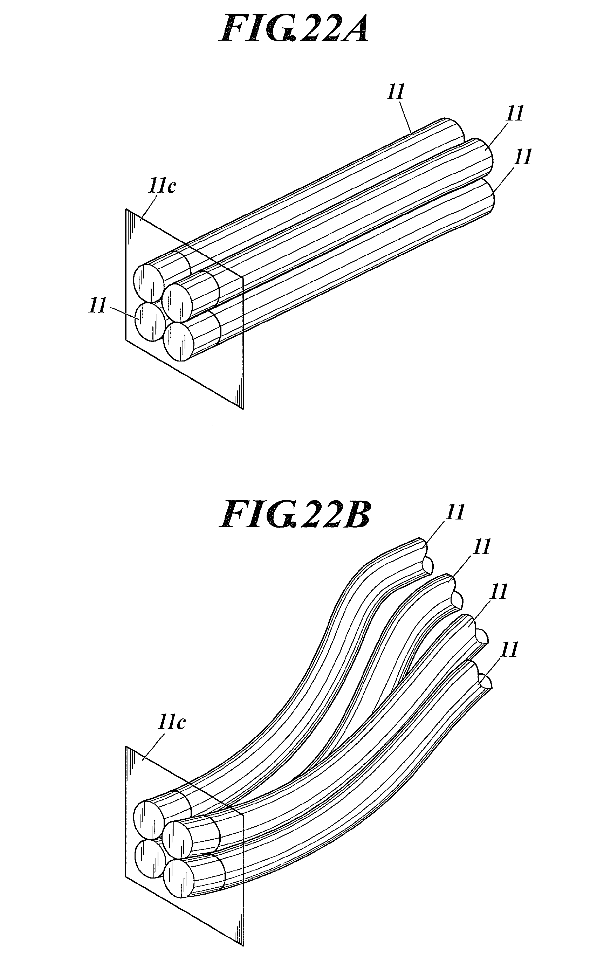

When a plurality of illuminating light guiding optical fibers 11 are used, in order to prevent unevenness of irradiated light due to variability in forming the exit end face, it is useful to fix together the plurality of optical fibers 11, 11 and so on to be used as shown in FIG. 22A and FIG. 22B, and to align the exit end face to a same face 11c by collectively performing grinding processing. Specifically, it is possible to obtain a more even exit end face by twisting together and fixing the optical fibers 11, 11 and so on as shown in FIG. 22B and then performing grinding processing.

### (Form of Holding Lens and Optical Fiber)

Here the form of holding the lens and the optical fiber is further described.
All of the above described lenses can be a target of the fixing configuration and fixing method of the lens described here.
The above described lenses either receive light guided by the optical fiber or guide the received light to the optical fiber. Therefore it is necessary to accurately position and fix the lens with respect to the optical fiber.
Therefore, a configuration which fixes the optical fiber and the lens to each other through one or a plurality of components is employed.

For example, in the configuration shown in FIG. 17A and FIG. 17B, a light guiding lens 36 is provided on the tip end side of the transmitting light optical fiber 9 and the receiving light optical fiber 10.
The transmitting light optical fiber 9 and the receiving light optical fiber 10 shown in FIG. 17A and FIG. 17B are directly held by the ferrule 50. As shown in FIG. 17B, the ferrule 50 is inserted and fixed in a hole section of a rear end section of the holder 51. The light guiding lens 36 is fixed in the front end section of the holder 51.
Therefore, according to the above configuration, the optical fibers 9 and 10 and the light guiding lens 36 are fixed to each other through a ferrule 50 and holder 51.
The antifouling hood 37 is connected and fixed to the front end section of the holder 51.

As the holder 51, a member which is formed by emitting material including biocompatibility such as liquid crystal polymer is applied. The holder 51 holds the illuminating light guiding optical fiber 11 and the ferrule 50 holding the transmitting light optical fiber 9 and the receiving light optical fiber 10. An adhesive including biocompatibility can be applied to fixing the illuminating light guiding optical fiber 11 and ferrule 50 to the holder 51.
As described above, by configuring the probe tip end section by applying the ferrule 50 and the holder 51, even if there is a change in the specification of the optical fibers 9 and 10 due to reasons such as change in optical design, reducing costs, etc., as long as the outer shape (at least the outer diameter) of the ferrule 50 is not changed, measures can be easily taken.
The ferrule 50 is inserted and fixed in the hole section of the rear end section of the holder 51. However, it is possible to reduce the variation in accuracy of assembly as much as possible by setting the ferrule 50 in the hole section and positioning by striking the base of the hole section by the tip end of the ferrule 50.

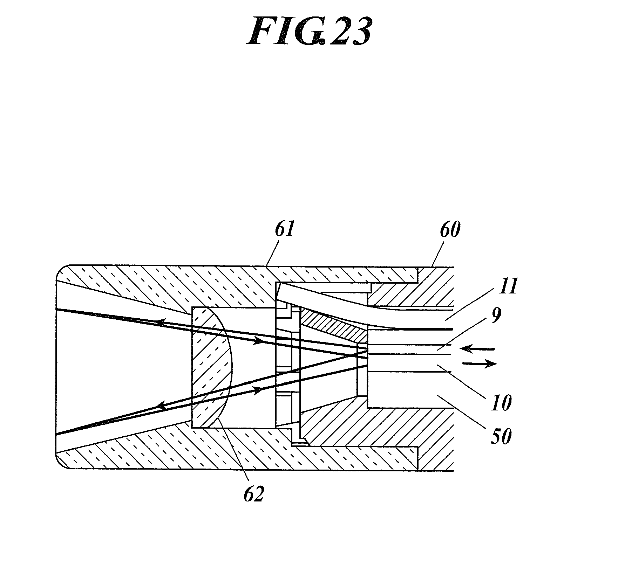

The light guiding lens 36 can be fixed to the optical fibers 9 and 10 through the antifouling hood 37, holder 51 and the ferrule 50 by fixing the light guiding lens 36 in the antifouling hood 37. The configuration corresponding to the above is shown in FIG. 23. In the configuration shown in FIG. 23, a holder 60 holds the illuminating light guiding optical fiber 11 and the ferrule 50 holding the transmitting light optical fiber 9 and the receiving light optical fiber 10. An antifouling hood 61 is joined and fixed to the front end section of the holder 60. The light guiding lens 62 is fixed in the antifouling hood 61.
It is preferable to perform the following steps in assembly of the configuration shown in FIG. 23.
The antifouling hood 61 and the lens 62 are assembled and test including test item of whether or not desired assembly accuracy is achieved is performed on the assembly of the antifouling hood 61 and the lens 62.
Separately, the ferrule 50 already holding the optical fibers 9 and 10, the illuminating light guiding optical fiber 11 and the holder 60 are assembled, and test including test item of whether or not desired assembly accuracy is achieved is performed on the assembly of the ferrule 50, the illuminating light guiding optical fiber 11 and the holder 6.
Then, the holder 60 and the antifouling hood 61 are connected to combine the above two intermediate assemblies and further assembly is performed. With this, test including test item of whether or not desired assembly accuracy is achieved is further performed on the assembled assembly.
According to the above, the probe can be configured with preferable efficiency and accuracy. It is preferable that a testing step is included for each intermediate assembly as described above for a probe other than the configuration shown in FIG. 23.

In the configuration shown in FIG. 14, a holding member 31 is applied for fixing the illuminating light guiding optical fiber 11 and the illuminating lens 30. In other words, the component between the illuminating light guiding optical fiber 11 and the illuminating lens 30 to fix the above is the holding member 31.

As described above, a component is applied between the optical fiber and the lens in order to accurately position and fix the lens to the optical fiber. Among the above described lenses, even where the component is not particularly illustrated, it is preferable to apply a component between the optical fiber and the lens to fix the above and to accurately determine the relative position of the above.
A preferable embodiment of a configuration of fixing a lens and the method of fixing the lens to the component which is between the optical fiber and the lens is disclosed below.

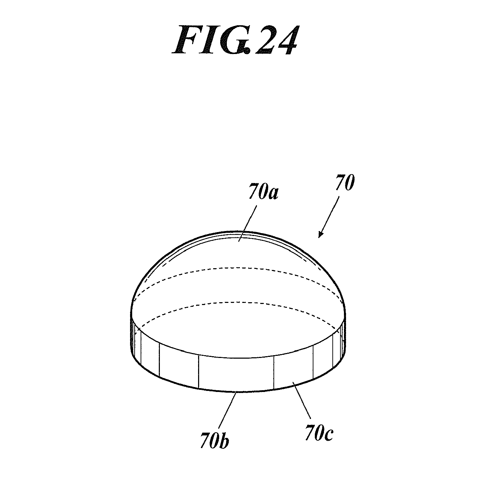

First, a circumferential wall section 70c formed by a cylinder face with the lens optical axis as the center axis as in the plane convex lens 70 shown in FIG. 24 is provided on the lens.
In FIG. 24, the plane convex lens 70 includes a convex face section 70a formed in a hemisphere shape, a plane section 70b formed in a substantial plane facing the convex face section 70a, and a circumferential wall section 70c formed between the convex face section 70a and the plane section 70b and formed by the cylinder face with the optical axis of the lens 70 as the center axis. The material of the plane convex lens 70 can be resin or glass. Since the circumferential wall section 70c is formed by a the cylinder face with the optical axis of the lens 70 as the center axis, the optical axis of the lens 70 can be mechanically determined by the circumferential wall section 70c, and this can be used to accurately position and fix the optical axis of the lens 70.
Moreover, by providing the circumferential wall section 70c, it is possible to handle the lens 70 by holding the circumferential wall section 70c. Therefore, it is possible to prevent the lens face from scratching and fouling and it is possible to prevent damage of the lens 70.

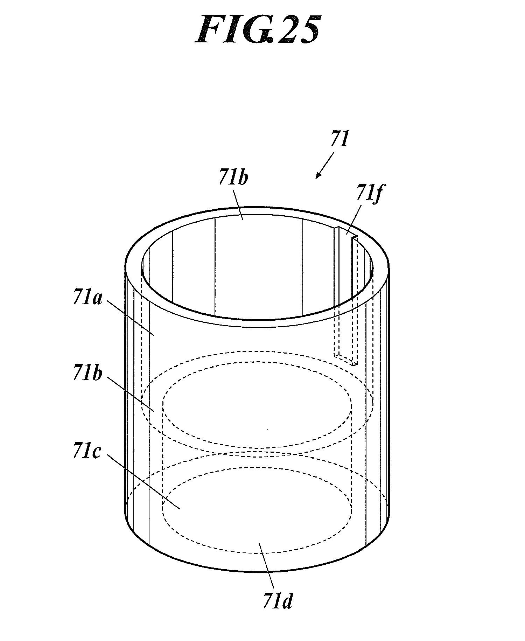

An example of fixing the above lens 70 in a cylinder shaped member 71 shown in FIG. 25 is described.
A first hollow section 71a and a second hollow section 71c pass through the cylinder shaped member 71. An outside opening section of the first hollow section 71a is to be 71b, and an outside opening section of the second hollow section 71c is to be 71d. The inner diameter of the first hollow section 71a is larger than the second hollow section 71c, and a step between the first hollow section 71a and the second hollow section 71c functions as a lens receiving section 71e which holds the circumferential border section of the plane section 70b of the lens 70. A groove section 71f for dripping adhesive is formed on an inner circumferential wall of the first hollow section 71a.
As described above, as a component to fix the lens, an inner configuration for fixing the lens where a cylinder shaped hollow with a relatively large diameter (corresponding to the first hollow section 71a) and a cylinder shaped hollow with a relatively small diameter (corresponding to the second hollow section 71c) are provided connected with the same axis and a step is provided circularly. The other configuration such as outer shape, etc. of the cylinder shaped member 71 can be any configuration. The above described antifouling hood 61, etc. is applied as the component corresponding to the cylinder shaped member 71 and the outer shape, etc. is designed with variety.

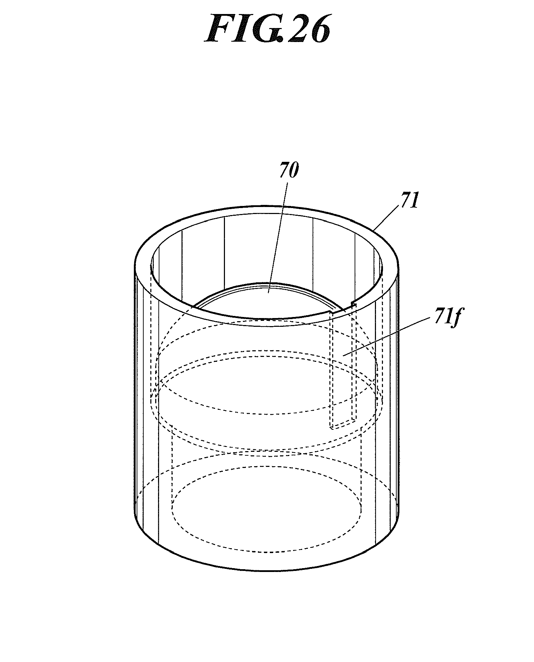

A lens 70 is inserted in the first hollow section 71a from the opening section 71b of the large diameter side of the cylinder shaped member 71, and the circumferential border section of the plane section 70b of the plane convex lens 70 is provided in contact with the lens receiving section 71e.
As shown in FIG. 25, the above described groove section 71f is formed from the opening section 71b, and is formed to a predetermined depth separated to the opening section 71b side from the lens receiving section 71e. As shown in FIG. 26, when the circumferential border section of the plane section 70b of the plane convex lens 70 is placed in contact with the lens receiving section 71e, the inner side closing end of the groove section 71f is deeper than the upper end of the circumferential wall section 70c, and is provided in a position shallower than the lens receiving section 71e, and therefore the lower end of the circumferential wall section 70c. Here, it is suitable that the inner side closing end of the groove section 71f is in a position substantially middle of the upper end and the lower end of the circumferential wall section 70c.

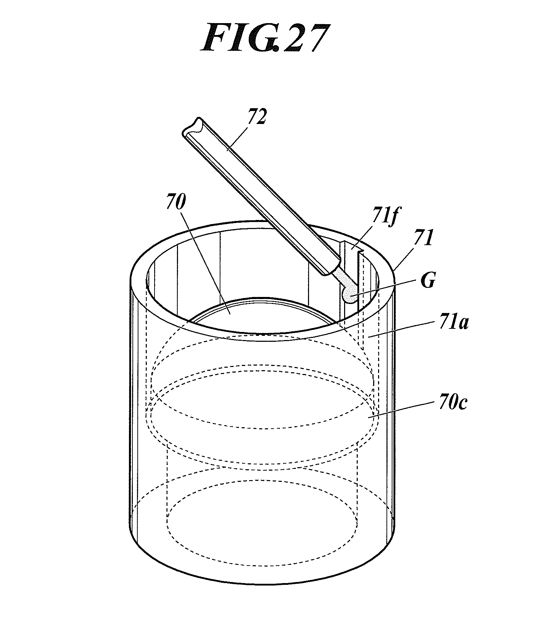

As described above, when the lens 70 is provided in the first hollow section 71a and positioned in the center, as shown in FIG. 27, a nozzle 72 of an adhesive dispenser is brought near the groove section 71f to eject adhesive G, and a predetermined amount of the adhesive G is dripped in the groove section 71f. The dripped adhesive G is flown evenly between the circumferential wall section 70c of the plane convex lens 70 and the inner circumferential wall of the first hollow section 71a of the cylinder shaped member 71 and the adhesive is filled so that the above are connected. By providing a circumferential wall section 70c, the surface area of the adhered lens 70 increases and the adhesive properties is enhanced.

It is preferable that the dripped amount of adhesive G is an amount where the adhesive G is filled to a height about half of the circumferential wall section 70c. If the amount of the adhesive G is small, the strength of adhesion is reduced and a difference in strength of adhesion between each of the merchandise easily occurs. Consequently, this is not preferable with respect to performance and quality. If the amount of the adhesive G is large, there is a possibility that the adhesive G is applied to the optical face (in this case, specifically the convex face section 70a). There is also a possibility that the stress due to hardening of the adhesive G becomes large, which causes deformation of the lens 70.

As the adhesive G, with the condition of being biocompatible, a thermal hardening type, an optical hardening type or a hybrid type of the above can be applied. After filling, heat or light is irradiated by a device not shown to harden the adhesive G.

By providing a groove section 71f which guides the dripped adhesive G to the connecting section, the spreading of the adhesive G can be prevented.

Moreover, if the groove section 71f reaches the lens receiving section 71e, there is a possibility that the adhesive G flows out to the plane section 70b of the plane convex lens 70. Moreover, bubbles may be generated in the adhesive G, and there is a possibility that the appearance is flawed and the connection is not sufficient. Therefore, as shown in FIG. 26, it is preferable that the groove section 71f does not reach the lens receiving section 71e, and terminates at about the middle of the circumferential wall section 70c of the plane convex lens 70.

Moreover, if a plurality of groove sections 71f is provided, there is a possibility that bubbles are generated in a portion where adhesive G flown from two groove sections 71f meet. Therefore, it is preferable that the groove section 71f is one.

According to the above embodiment, it is described that the optical fiber irradiates excitation light to the observation target region and receives fluorescence which occurs due to the excitation light. However, the optical fiber can receive scattering light or raman scattering light which occurs due to the excitation light. Even in the above examples, it is possible to perform diagnosis of disease state such as degeneration and cancer of biological tissue.
In the above embodiment, as material of the holding member applied for holding the optical fiber, sintered zirconia is biocompatible and can be applied. However, the material is not limited to the above as long as the material is biocompatible, and other material such as resin, metal, etc. can be applied. Specifically, it is preferable to apply resin material with biocompatible properties because the shape of the holding section, etc. of the optical fiber can be molded with high accuracy.
In addition to the above described configuration, a mechanism to clean the tip end of the endoscope main body 1 can be provided in the probe 4. In order to realize the above, for example, a pump is provided in the base unit, etc. a liquid supplying tube is passed through the probe 4 from the pump to be in communication with an ejecting opening provided in the probe 4. It is preferable that the ejecting direction from the ejecting opening is set with a suitable angle such as a side direction, a diagonally rear direction, etc. so that the liquid can be ejected on a tip end face of the endoscope main body 1.

### Industrial Applicability

The present invention can be used for observation of biological tissue for the purpose of medical diagnosis.

### Description of Reference Numerals

1 endoscope main body
1a electronic camera
1b illumination of endoscope main body
1c endoscope channel
1d inserting opening
1e tip end opening
2 endoscope processor
3 endoscope display monitor
4 probe
4a tube
5 base unit
6 display monitor
7 operation/input device
8a image signal cable
8b image signal cable
9 transmitting light optical fiber
10 receiving light optical fiber
11 illuminating light guiding optical fiber
12a light guiding lens
12b light guiding lens
13 light emitting material piece
14 light source
15 optical fiber
16 light emitting diode
17 power source line
18 power source
19 holding member
19a cutout section
20 holding member
20a cutout section
21 light guiding lens
22 light guiding lens
22a flange section
23 light guiding lens
23a flange section
23b circumferential groove
24 light guiding lens
24a flange section
24b groove
24c inclined face
25 light guiding lens
25a flange section
25b illuminating lens
26 light guiding lens
26a flange section
26b illuminating lens
27 light guiding lens
28 holding member
29 holding member
30 illuminating lens
31 holding member
31a inserting hole
31b latching section
32 ball lens
33 cap member
34 light guiding lens
34a excitation light
34b space
35 antifouling hood
35a holding hole
36 light guiding lens
36b groove
37 antifouling hood
37a holding hole
38 light guiding lens
39 holding member
39a groove
40 antifouling hood
41 multi-lumen tube
41a hole
42 optical fiber
43 concave lens
44 convex lens
50 ferrule
51 holder
60 holder
61 antifouling hood
62 light guiding lens
70 plane convex lens
71 cylinder shaped member

## Claims

1. A probe inserted through an endoscope channel comprising at least:
a transmitting light optical fiber; and
a receiving light optical fiber,
wherein excitation light guided by the transmitting light optical fiber is irradiated to an observation target region of biological tissue and emitted light emitted from the observation target region due to the excitation light is received to be guided to a base end side of the probe by the receiving light optical fiber,
the prove further comprising an illuminating section to be used in imaging by an imaging section of an endoscope main body.

2. The probe of claim 1, further comprising a light guiding lens which guides the excitation light and the emitted light to a tip end side of the transmitting light optical fiber and the receiving light optical fiber.

3. The probe of claim 2, wherein the light guiding lens is a light condensing lens.

4. The probe of claim 2, wherein the light guiding lens is a collimated lens.

5. The probe of claim 2, further comprising a tube with water shielding properties to form an outer circumferential wall, wherein a tip end opening of the tube is sealed to be waterproof by the light guiding lens.

6. The probe of claim 1, further comprising a tube with water shielding properties to form an outer circumferential wall, wherein a tip end opening of the tube is sealed to be waterproof by a cover member.

7. The probe of claim 1, further comprising a tube with water shielding properties to form an outer circumferential wall, wherein a tip end face of the transmitting light optical fiber and the receiving light optical fiber is exposed to a probe tip end and a space between the tip end section of the transmitting light optical fiber and the receiving light optical fiber and the tube is sealed to be waterproof.

8. The probe of any one of claims 1 to 7, further comprising a light emitting diode provided at a probe tip end section as the illuminating section.

9. The probe of any one of claims 1 to 7, further comprising illuminating light guiding optical fiber which guides illuminating light from an illuminating light source to a probe tip end section as the illuminating section.

10. The probe of any one of claims 1 to 7, further comprising as the illuminating section, a light emitting material piece which is provided at a probe tip end section and which emits white color fluorescence by excitation, and a section which irradiates excitation light to the light emitting material piece.

11. The probe of claim 10, further comprising a light emitting diode provided adjacent to the light emitting material piece as the section which irradiates excitation light to the light emitting material piece.

12. The probe of claim 10, further comprising optical fiber which guides excitation light from a light source to the light emitting material piece as the section which irradiates excitation light to the light emitting material piece.

13. The probe of claim 2, further comprising illuminating light guiding optical fiber which guides illuminating light from an illuminating light source to a probe tip end section as the illuminating section, wherein the illuminating light guiding optical fiber is provided so that an output light end faces an outer circumferential section of the light guiding lens to allow the guided illuminating light to enter the outer circumferential section of the light guiding lens.

14. The probe of claim 2, wherein a flange section is formed outside the effective diameter of the light guiding lens, and the probe further comprises illuminating light guiding optical fiber which guides illuminating light from an illuminating light source to a probe tip end section as the illuminating section, wherein the illuminating light guiding optical fiber is provided so that an output light end faces the flange section to allow the guided illuminating light to enter the flange section.

15. The probe of claim 2, wherein a circumferential groove is formed in a circular shape in the flange section; and the output light end section of the illuminating light guiding optical fiber is inserted in the circumferential groove.

16. The probe of either one of claim 14 or claim 15, wherein a diffusing section which diffuses illuminating light is formed in the flange section and the output light end of the illuminating light guiding optical fiber is provided to be in contact with the flange section.

17. The probe of either one of claim 14 or claim 15, further comprising a diffusing plate which is provided between the flange section and the output light end of the illuminating light guiding optical fiber and which diffuses illuminating light, wherein the output light end of the illuminating light guiding optical fiber is provided to be in contact with the diffusing plate.

18. The probe of any one of claims 14 to 17, wherein the output light end of the illuminating light guiding optical fiber is provided so as to be inclined outward and a facing face of the flange section facing the output light end is formed so as to be inclined inward.

19. The probe of any one of claims 14 to 18, wherein an illuminating lens which corresponds to each one of the illuminating light guiding optical fiber and which diffuses the illuminating light is formed in the flange section.

20. The probe of claim 19, further comprising a plurality of the illuminating lenses wherein the optical axes of the illuminating lenses are provided on a same concentric circle.

21. The probe of claim 2, wherein an outer shape of the light guiding lens is formed in a D shape where a portion is missing from a round shape and an electric line or optical fiber composing the illuminating section is passed through a space corresponding to the missing section.

22. The probe of any one of claims 1 to 7, further comprising a holding member which holds the optical fiber composing the illuminating section, the transmitting light optical fiber and the receiving light optical fiber.

23. The probe of claim 22, wherein the optical fiber composing the illuminating section is latched to a cutout section formed on an outer circumference of the holding member.

24. The probe of claim 23, wherein the cutout section is formed in a tapered shape.

25. The probe of claim 22, wherein the optical fiber composing the illuminating section is inserted through a through hole formed on the holding member and latched.

26. The probe of claim 9, wherein illuminating lenses which correspond to each one of the illuminating light guiding optical fiber and which diffuse illuminating light guided by the illuminating light guiding optical fiber are provided by separate independent components.

27. The probe of claim 26, further comprising a light guiding lens which guides the excitation light and the emitted light to a tip end side of the transmitting light optical fiber and the receiving light optical fiber, wherein a flange section is formed outside the effective diameter of the light guiding lens, and the illuminating light guiding optical fiber is inserted through a through hole or groove formed in the flange section and latched.

28. The probe of claim 26, wherein the illuminating lens is composed from a plurality of ball lenses with respect to one of the illuminating light guiding optical fiber.

29. The probe of any one of claims 1 to 7, further comprising an illuminating light guiding optical fiber which guides illuminating light from an illuminating light source to a probe tip end section as the illuminating section and an antifouling hood provided at the probe tip end, wherein a tip end section of the illuminating light guiding optical fiber extending in a tip end direction than the transmitting light optical fiber and the receiving light optical fiber is held by the antifouling hood.

30. A diagnosis device comprising:
a probe described in any one of claims 1 to 29;
a base unit connected to a rear end of the probe; and
an operation input device connected to the base unit,
wherein the base unit controls turning on and off of the illuminating section based on an operation signal from the operation input device.

31. The diagnosis device of claim 30, wherein the base unit is provided with a spectroscopic analysis section which performs spectroscopic analysis of light taken in from the receiving light optical fiber and the analysis target of the spectroscopic analysis section is light taken in from the receiving light optical fiber when the illuminating section is turned off.

32. The diagnosis device of claim 31, wherein the base unit is provided with an interface which inputs an image signal of an endoscope and an image composite section which overlaps and combines an image of the endoscope and an analysis result by the spectroscopic analysis section.

33. The diagnosis device of claim 30, wherein the base unit includes an interface which inputs an image signal of an endoscope and a light modulating section which modulates light of the illuminating section based on an image signal of the endoscope.

34. A method for using a diagnosis device of any one of claims 30 to 33 wherein the probe is inserted through the endoscope channel of the endoscope main body and after the illumination of the endoscope main body is turned off, the illuminating section is used as illumination when imaging is performed by the imaging section of the endoscope main body.
